# EUROPEAN PATENT APPLICATION

(11) **EP 4 449 891 A1**
(43) Date of publication of application: **23.10.2024**
(21) Application number: 21968067.5
(22) Date of filing: 14.12.2021
(51) Int. Cl.: A24B 13/00

(54) **ORAL PRODUCT CONTAINING LOW MOLECULAR WEIGHT ALGINIC ACID COMPOUND AND COMPOSITION FOR SAID PRODUCT**

(71) Applicant: Japan Tobacco Inc., Tokyo 105-6927 (JP)
(72) Inventor: HAMAMOTO, Takeshi, Tokyo 130-8603 (JP); ENDO, Shoko, Tokyo 130-8603 (JP)
(74) Representative: Hoffmann Eitle
(86) International application number: PCT/JP2021/046065
(87) International publication number: WO 2023/112150

(57) **Abstract**

Provided is a composition for an oral product, the composition comprising an alginic acid compound having a weight-average molecular weight of 10,000-900,000 and selected from the group consisting of alginic acid, salts thereof, esters thereof, and combinations of the foregoing.

## Description

### TECHNICAL FIELD

The present invention relates to an oral product containing a low molecular weight alginic acid compound and a composition for the product.

### BACKGROUND ART

Oral tobacco products like snus are known (e.g., PTL 1). The user uses the product by placing it in the oral cavity. Meanwhile, alginic acid is known as a functional material used in, for example, food and beverages. For instance, in PTL 2, a beverage containing alginic acid is disclosed. The publication argues that the beverage limits the increase in blood glucose and insulin levels. In PTL 3, furthermore, an oral product containing an alginic acid salt is disclosed. The publication argues that the product enables the regulation of the rate of release of substances added to it.

### CITATION LIST

### PATENT LITERATURE

PTL 1: International Publication No. 2012/133365
PTL 2: Japanese Unexamined Patent Application Publication No. 6-7093
PTL 3: Japanese Unexamined Patent Application Publication (Translation of PCT Application) No. 2012-522765

### SUMMARY OF INVENTION

### TECHNICAL PROBLEM

Oral products have been disadvantageous in that the product during use has an unpleasant mouthfeel until saliva penetrates into the product. Under such circumstances, an object of the present invention is to provide an oral product that has a good mouthfeel during use.

### SOLUTION TO PROBLEM

The inventors formulated the conception of solving this problem by using an alginic acid compound selected from the group consisting of alginic acid, salts thereof, esters thereof, and combinations of the foregoing. To this end, the inventors conducted a preliminary study using commercially available alginic acid or its salt, but failed to adequately solve the problem. After reaching the findings that this issue is due to high molecular weights of alginic acid compounds, the inventors realized that the above problem can be solved by using alginic acid compounds with particular molecular weights. That is, the above problem is solved by the present invention described below.

### Aspect 1

A composition for an oral product, the composition containing an alginic acid compound having a weight-average molecular weight of 10,000-900,000 and selected from the group consisting of alginic acid, salts thereof, esters thereof, and combinations of the foregoing.

### Aspect 2

The composition according to aspect 1, wherein an amount of the alginic acid compound in the composition is 1-99% by weight.

### Aspect 3

The composition according to aspect 1 or 2, further containing a vegetable alkaloid.

### Aspect 4

An oral product containing the composition according to any of aspects 1 to 3.

### Aspect 5

An oral product comprising an exterior material and a main material, wherein:
the exterior material or the main material contains an alginic acid compound having a weight-average molecular weight of 10,000-900,000 and selected from the group consisting of alginic acid, salts thereof, esters thereof, and combinations of the foregoing.

### Aspect 6

The oral product according to aspect 5, wherein the exterior material contains the alginic acid compound.

### Aspect 7

The oral product according to aspect 4, wherein the oral product is in chewing tobacco, pouch, tablet, gum, film, or liquid form.

### Aspect 8

The oral product according to aspect 5 or 6, wherein the oral product is in pouch or gum form.

### Aspect 9

A method for manufacturing the composition according to any of aspects 1 to 3, the method including:
a step of preparing the alginic acid compound by heating a raw-material alginic acid compound having a higher molecular weight than the alginic acid compound for 50 minutes or more at 95-100°C under normal pressure.

### ADVANTAGEOUS EFFECTS OF INVENTION

According to the present invention, an oral product that has a good mouthfeel during use can be provided.

### DESCRIPTION OF EMBODIMENTS

The present invention will now be described in detail. In the present invention, "X-Y" includes its limits, X and Y. An oral product is a product intended for the user to ingest its active ingredient through the oral mucosa via saliva while the product is within the oral cavity. An oral product does not require burning or device-assisted heating or atomization during its use.

### 1. Composition for Oral Product

### (1) Alginic Acid Compound

The composition for an oral product (hereinafter also simply referred to as "the composition") contains an alginic acid compound having a weight-average molecular weight of 10,000-900,000 and selected from the group consisting of alginic acid, salts thereof, esters thereof, and combinations of the foregoing.

Alginic acid is a linear-chain polysaccharide composed of two types of uronic acids, D-mannuronic acid (hereinafter also referred to as "M") and L-guluronic acid (hereinafter also referred to as "G"), and contains a block consisting solely of M-M bonds, a block consisting solely of G-G bonds, and a random block, in which M and G are randomly bound. Typically, alginic acid is manufactured from brown algae and includes salt or ester forms. In the present invention, alginic acid, salts thereof, and esters thereof are collectively referred to as "alginic acid compounds." Alginic acid compounds are manufactured by various methods; in the present invention, however, alginic acid compounds obtained by known methods can be used.

Alginic acid compounds commercially available to the public have a high molecular weight. In the present invention, therefore, it is required to reduce the molecular weight so that the weight-average molecular weight will fall within the range of 10,000-900,000. Known methods for molecular weight reduction include hydrolysis using acid, biodegradation using a breakdown enzyme, the method of heating the compound under increased pressure (e.g., PTL 2), and the method of heating the compound with pressure normalization (Kazuyuki Iwata et al, the Journal of Home Economics of Japan, vol. 48, No. 9, p. 803

(1997)). In the present invention, molecular weight reduction by heating a raw-material alginic acid compound under normal pressure is preferred from viewpoints such as device simplicity and safety during manufacture. The temperature is preferably 95-100°C. The duration of heating, furthermore, is preferably 50 minutes or more. Optionally, the alginic acid compound may be heated in the presence of an organic acid. The organic acid is preferably an acid acceptable as food, such as citric acid, malic acid, lactic acid, or acetic acid. Its amount is adjusted as appropriate according to the desired molecular weight.

The weight-average molecular weight of the alginic acid compound used in the present invention is 10,000-900,000 as stated above. Preferably, however, the weight-average molecular weight is 12,500-60,000, more preferably 15,000-50,000. The weight-average molecular weight is measured by gel permeation chromatography (GPC).

When the alginic acid compound is an alginic acid salt, examples of counterions include Na⁺, K⁺, Ca²⁺, or Mg²⁺. When the alginic acid compound is an alginic acid ester, it may be, for example, the propylene glycol ester.

The amount of the alginic acid compound in the composition is preferably 1-99% by weight, more preferably 5-50% by weight.

The alginic acid compound, similar to high molecular weight alginic acid compounds, is expected to have the effect of limiting the increase in blood glucose and insulin levels during a glucose load. Oral products containing the composition according to the present invention for an oral product, therefore, may be used during or after meals in an embodiment.

### (2) Vegetable Alkaloid

The composition may contain a vegetable alkaloid. Vegetable alkaloids are basic organic compounds containing nitrogen atoms. Examples of vegetable alkaloids include substances like nicotine, caffeine, theobromine, and capsaicin. Examples of forms in which the substance is blended in the composition include a plant that contains the substance and a reconstituted material obtained by processing such a plant, a plant-derived extract containing the substance, or a raw material containing the substance stabilized with, for example, a carrier or salt.

The vegetable alkaloid is preferably nicotine, a nicotine-containing raw material, or a combination thereof. A nicotine-containing raw material refers to a raw material containing nicotine, such as a nicotine salt or stabilized nicotine. The same applies to raw materials containing other vegetable alkaloids, such as caffeine, theobromine, and capsaicin. An example of stabilized nicotine is a nicotine carrier, such as nicotine supported on an ion-exchange resin. An example of an ion-exchange resin is a weakly acidic cation-exchange resin. An ion-exchange resin with nicotine supported on it can specifically be a resin complex that contains, for example, 10% by weight or more and 20% by weight or less nicotine, referred to as nicotine polacrilex. The ion-exchange resin used in nicotine polacrilex is a weakly acidic cation-exchange resin. When nicotine polacrilex is used, its amount added in relation to the composition is typically 0.5% by weight or more, preferably 1.0% by weight or more, more preferably 2.0% by weight or more. For savor reasons, however, the amount of nicotine polacrilex added in relation to the composition is typically 15.0% by weight or less, preferably 12.0% by weight or less, more preferably 10.0% by weight or less.

Moreover, a nicotine-containing raw material may be, for example, a tobacco material containing a tobacco powder obtained by grinding tobacco leaves. The tobacco powder may include constituents such as shreds, fine powder, or fibers of the lamina of dried tobacco leaves and is prepared by, for example, the method described below. In the present invention, tobacco leaves may include the lamina, the stem, or the root. Besides a tobacco powder basically obtained from the lamina of tobacco leaves, the tobacco material may contain components derived from the midrib or root of tobacco leaves.

The particle diameter of the tobacco powder is not limited. In order for the composition to fit well inside the oral cavity and feel better during use and for good release of a flavor component contained in the tobacco powder into the oral cavity, however, it is preferred that the tobacco powder be one that has passed through a 1.2-mm mesh. More preferably, the tobacco powder is one that has passed through a 1.0-mm mesh.

The tobacco species that serves as a raw material for the tobacco powder is not particularly limited. Examples include species within the *Nicotiana* genus, such as the flue-cured and burley cultivars of *Nicotiana tabacum* and the Brazilian cultivar of *Nicotiana rustica.* For the tobacco material and tobacco leaves, described later herein, too, the same species as these can be used.

The tobacco powder is preferably prepared as follows. First, a base is added to and mixed with a tobacco powder obtained by grinding tobacco leaves. The base that is added can be, for example, potassium carbonate or sodium carbonate, and it is preferred to add it as an aqueous solution. A pH-adjusting agent like sodium dihydrogenphosphate, furthermore, may be added, for example for the stabilization of nicotine during the manufacture of an oral pouch product. The pH of the mixture after the addition of the base is preferably adjusted to 8.0-9.0. The percentage of the tobacco powder in this mixture is preferably 60-90% by weight.

After the addition of the base, heating is performed, for example for 0.5-3 hours, preferably 0.8-2 hours, for example under conditions under which the mixture temperature will be 65-90°C, preferably the mixture temperature will be 70-80°C. Through this, the sterilization of the tobacco powder is carried out. The heating can be performed by either one or both of heating by steam injection and heating with a jacket. The pH of the mixture after heating is preferably 8.0-9.0, and the water content of the mixture after heating is preferably 10-50% by weight.

After heating, in an embodiment, the resulting processed tobacco powder may be subjected to drying treatment by performing heating with a jacket alone, optionally after stopping steam injection. Then the powder may be cooled at approximately 15-25°C for approximately 1 hour.

When a tobacco material containing a tobacco powder is used, its amount added in relation to the composition is typically 0.001% by weight or more, preferably 0.01% by weight or more, more preferably 0.05% by weight or more. For savor reasons, however, the amount of the material added in relation to the composition is typically 90% by weight or less, preferably 80% by weight or less, 70% by weight or less, 45% by weight or less, 40% by weight or less, or 30% weight or less.

A nicotine-containing raw material may be a nicotine-containing extract obtained through extraction from a nicotine-containing substance, such as tobacco leaves.

Of the forms described above, the use of a nicotine carrier is particularly preferred from the viewpoints of appropriate supply of nicotine and the ease of handling. In addition, when a tobacco powder is added, the color of the composition and the oral product usually tends to be the color of the tobacco leaves. When a colorless nicotine-containing compound is used, however, it is possible to provide a white composition and oral product. For those users who like white oral products, such a form is an advantage. One raw material as described above may be used alone, or two or more may be used in combination.

The percentage of total nicotine in the composition is not limited, but for palatability to users, it is typically 0.1-20.0% by weight. When a nicotine-containing raw material is used as a vegetable alkaloid, therefore, the amount of the raw material is adjusted so that the total nicotine percentage will fall within this range. When the nicotine is present as ions, this percentage is a percentage as nicotine ions. The percentage of nicotine in the composition can be measured by, for example, gas chromatography-mass spectrometer (GC-MS) or liquid chromatography (LC, UV detection).

Ingredients other than those described above are selected as appropriate according to the form of the oral product, which is the final form. In the following, ingredients used primarily in a composition for an oral pouch product will be described.

### (3) Substrate

The composition preferably contains a substrate. The substrate is not particularly limited, and examples include polysaccharides or porous structures capable of adsorbing and holding water. Specifically, the substrate is preferably one or more selected from the group consisting of cellulose, microcrystalline cellulose (MCC), spherical cellulose, and porous cellulose. More preferably, the substrate is cellulose in particular, for flexibility in the adjustment of the bulk density of the composition and because it displays a white color. One such substance may be used alone, or two or more may be used in combination in any proportions.

The percentage of the substrate in the composition (when the composition contains two or more substrates, their total percentage) is not limited. In order that a quality improvement will be sought by reducing the exudation of water during manufacture or product storage and that an appearance desirable to users will be imparted through the enhancement of the whiteness of the product, however, the percentage is typically 10% by weight or more. Preferably, the percentage is 15% by weight or more, more preferably 20% by weight or more. The upper limit to this percentage, furthermore, is not limited, but in view of the limits on how much other raw materials can be contained, the percentage is typically 70% by weight or less. Preferably, the percentage is 68% by weight or less, more preferably 65% by weight or less.

### (4) Sugar Alcohol

The composition preferably contains a sugar alcohol. The type of sugar alcohol is not particularly limited, and examples include xylitol, maltitol, erythritol, sorbitol, mannitol, and lactitol. Of these, maltitol is preferred from the viewpoint of imparting a good savor. One such substance may be used alone, or two or more may be used in any proportions.

The percentage of the sugar alcohol in the composition (when the composition contains two or more sugar alcohols, their total percentage) is not limited, but for savor adjustment reasons, it is typically 1% by weight or more. Preferably, the percentage is 5% by weight or more, more preferably 10% by weight or more. Its upper limit, furthermore, is typically 80% by weight or less, preferably 70% by weight or less, more preferably 60% by weight or less.

### (5) Polyglycerine Fatty Acid Ester

The composition preferably contains a polyglycerine fatty acid ester. The degree of polymerization of glycerine in the polyglycerine fatty acid ester is preferably 2-10. The polyglycerine fatty acid ester functions as an emulsifier. Incorporating a polyglycerine fatty acid ester, therefore, ensures that a state in which the ingredients in the composition are evenly mixed will be held. The flavor component can be kept in a stable state, which helps achieve a good flavor of the composition. The polyglycerine fatty acid ester, furthermore, imparts moderate viscosity to the composition, allowing its ingredients to be bound together. The ester, therefore, retards the stiffening and drying of the composition, helping achieve good characteristics, such as feel during use and flavor. In addition to these, the ester helps reduce the scattering of the composition during its packing in the pouches or other exterior material even when the composition is of dry type, in which the amount of water (the water content) is small. Overall, incorporating a polyglycerine fatty acid ester in the composition leads to improvements in efficiency in the manufacture of the oral product, such as operational efficiency and production yield.

Polyglycerine fatty acid esters are fatty acid esters of a dehydration condensate of glycerine. The degree of polymerization of the glycerine is typically two or greater and may be three or greater, and typically is ten or less and may be eight or less.

Fatty acid ester groups (RCOO- groups) in a polyglycerine fatty acid ester are derived from a fatty acid. The fatty acid is not limited; it may be a saturated fatty acid or may be an unsaturated fatty acid. The number of carbons in the fatty acid, furthermore, is typically 10 or more for a good flavor and manufacturing efficiency reasons. Preferably, the number of carbons is 12 or more, more preferably 14 or more, even more preferably 16 or more. Typically, furthermore, the number of carbons is 30 or fewer, preferably 26 or fewer, more preferably 22 or fewer, even more preferably 20 or fewer. The fatty acid may have a substituent or may be unsubstituted.

The number of fatty acid ester groups that one molecule of the polyglycerine fatty acid ester has is not limited as long as the polyglycerine fatty acid ester has a structure that allows it to serve a function as an emulsifier; it can be selected as appropriate according to the degree of polymerization of glycerine and the number of glycerine-derived hydroxyl groups. A structure that allows the polyglycerine fatty acid ester to serve a function as an emulsifier is a structure having both of a fatty acid moiety, which serves as a lipophilic group, and a polyhydric alcohol moiety, which serves as a hydrophilic group. Specifically, the number of fatty acid ester groups per molecule of the polyglycerine fatty acid ester only needs to be one or more in general. The number of glycerine-derived hydroxyl groups, furthermore, only needs to be one or more.

The degree of polymerization of glycerine and the type and number of fatty acid ester groups in the polyglycerine fatty acid ester can be chosen in any combination from the options presented above. More specifically, the alcohol component of the polyglycerine fatty acid ester can be diglycerine, triglycerine, tetraglycerine, pentaglycerine, hexaglycerine, heptaglycerine, octaglycerine, nonaglycerine, or decaglycerine. The acid component of the polyglycerine fatty acid ester can be a fatty acid such as lauric acid, myristic acid, palmitic acid, stearic acid, oleic acid, linoleic acid, or α-linolenic acid. The polyglycerine fatty acid ester, furthermore, can be, for example, a monoester, diester, triester, tetraester, or pentaester. One ester may be used alone as a polyglycerine fatty acid ester, or two or more may be used in combination in any proportions.

For a good flavor and the ease of operation during manufacture, the polyglycerine fatty acid ester is preferably one or more selected from diglycerine monofatty acid esters and decaglycerine fatty acid esters. A diglycerine monofatty acid ester is preferably selected from the group consisting of diglycerine monolaurate, diglycerine monomyristate, diglycerine monopalmitate, diglycerine monostearate, and diglycerine monooleate, more preferably is diglycerine monooleate. A decaglycerine fatty acid ester is preferably selected from the group consisting of decaglycerine laurates, decaglycerine myristates, decaglycerine palmitates, decaglycerine stearates, and decaglycerine oleates, more preferably is selected from the group consisting of decaglycerine monolaurate, decaglycerine monomyristate, decaglycerine monopalmitate, decaglycerine monostearate, and decaglycerine monooleate.

The percentage of the polyglycerine fatty acid ester in the composition (when the composition contains two or more polyglycerine fatty acid esters, their total percentage) is not limited, but for a good flavor to be obtained and for improved manufacturing efficiency, the percentage is typically 0.1% by weight or more. Preferably, the percentage is 0.2% by weight or more, more preferably 0.3% by weight or more, even more preferably 0.5% by weight or more. In order for moderate viscosity to be imparted to the composition, furthermore, the percentage of the polyglycerine fatty acid ester is typically 20.0% by weight or less. Preferably, the percentage is 15.0% by weight or less, more preferably 10.0% by weight or less, even more preferably 8.0% by weight or less.

The HLB value of the polyglycerine fatty acid ester is not limited, but for a good flavor to be obtained and for improved manufacturing efficiency, the HLB value is typically 6.0 or greater, preferably 7.0 or greater. In addition, the HLB value is typically 20.0 or less, preferably 18.0 or less, more preferably 16.0 or less.

### (6) Release Agent

The composition preferably contains a release agent. The release agent mitigates troubles like the adhesion of ingredients to manufacturing devices, such as a mixer and a kneader, during the manufacture of the composition or the adhesion of ingredients, for example to a packer, during the packing of the composition in the pouches or other exterior material, helping improve manufacturing efficiency. The release agent, furthermore, reduces caking (aggregation or solidification of the materials constituting the composition) by decreasing the adhesiveness between the materials and helps reduce the stickiness of the composition as well. As a result, the appearance, feel during use, such as feel in the mouth, flavor, etc., of the oral product including the composition improve. In addition, the release agent allows for the reduction of the leakage of the composition from the exterior material, helping improve the characteristics of the oral product.

The release agent is not limited as long as it delivers the above advantages. Examples of release agents include compounds such as particulate silicon dioxide, magnesium oxide, calcium silicate, magnesium silicate, calcium phosphate, calcium stearate, and magnesium stearate. Of these, it is preferred that the release agent be silicon dioxide because it is superior in the advantages described above and has little impact on flavor. One release agent may be used alone, or two or more may be used in combination in any proportions.

The release agent is preferably in particulate form so that it will deliver the above advantages sufficiently. For improved manufacturing efficiency, the average diameter of the particles is typically 20.0 µm or less. Preferably, the average particle diameter is 15.0 µm or less, more preferably 10.0 µm or less. The average particle diameter, furthermore, is typically 0.2 µm or more, preferably 0.3 µm or more, more preferably 0.4 µm or more, even more preferably 1.0 µm or more. In the present invention, the average diameter of release agent particles represents the particle diameter at a cumulative volume-based percentage of 50% (D50) in a particle size distribution determined through laser diffraction particle size distribution measurement. The laser diffraction particle size distribution measurement can be performed using a general-purpose device, such as "Mastersizer 3000," manufactured by Malvern Panalytical Ltd. In the present invention, furthermore, silicon dioxide having such an average particle diameter may be referred to as "particulate silicon dioxide."

The percentage of the release agent in the composition (when the composition contains two or more release agents, their total percentage) is not limited, but to ensure a good flavor and for the agent to deliver the above advantages sufficiently, the percentage is typically 0.05% by weight or more. Preferably, the percentage is 0.1% by weight or more, more preferably 0.5% by weight or more. The percentage, furthermore, is typically 3.0% by weight or less, preferably 2.5% by weight or less, more preferably 2.0% by weight or less.

### (7) Water

The percentage of water in (water content of) the composition is typically 5% by weight or more for the ease of manufacture of the composition. From viewpoints such as improving efficiency in the manufacture of the composition, improving the anticaking properties of the composition, and reducing the stickiness of the composition, furthermore, it is preferred that the lower limit to the percentage of water be 30% by weight or more, more preferably 45% by weight or more. The upper limit is typically 60% by weight or less, preferably 50% by weight or less. In addition, the percentage of water may be 40% by weight or less, may be 30% by weight or less, or may be 20% by weight or less. The percentage of water can be controlled by adjusting the amount of water added and providing heating treatment or drying treatment during the manufacturing stage. The percentage of water in the composition is controlled as appropriate according to the type of product (moist or dry). In the case of the moist type, for example, the percentage of water is typically 20-60% by weight, preferably 30-50% by weight. By contrast, in the case of the dry type, the percentage of water is typically 5-20% by weight, preferably 10-15% by weight.

The percentage of water in (water content of) the composition can be measured using a heat-drying moisture analyzer (e.g., HB 43-S: manufactured by METTLER TOLEDO). For the measurement, a sample is put into a predetermined container and heated to an attained temperature of 100°C. The measurement is concluded at the time when the amount of change becomes 1 mg or less per 60 seconds, and the water content is calculated from the measured weights before and after heating.

### (8) Others

Besides the foregoing, the composition may contain extra substances. Examples of extra substances include flavoring agents, pH-adjusting agents, sweeteners (excluding sugar alcohols), humectants, bitterness reducers, white coloring agents (excluding silicon dioxide), and emulsifiers (excluding polyglycerine fatty acid esters). The percentages of the substances are not limited; the amounts can be adjusted as appropriate according to the product design.

### 1) Flavoring Agent

Flavoring agents are not limited, and examples include menthol, leaf tobacco extract, natural vegetable flavoring agents (e.g., cinnamon, sage, herbs, chamomile, kudzu, sweet hydrangea leaves, clove, lavender, cardamom, caryophyllus, nutmeg, bergamot, geranium, honey essence, rose oil, lemon, orange, cinnamon bark, caraway, jasmine, ginger, coriander, vanilla extract, spearmint, peppermint, cassia, coffee, celery, cascarilla, sandalwood, cocoa, ylang-ylang, fennel, anise, licorice, St. John's bread, plum extract, and peach extract), saccharides (e.g., glucose, fructose, isomerized sugar, caramel, honey, and molasses), types of cocoa (powder, extract, etc.), esters (e.g., isoamyl acetate, linalyl acetate, isoamyl propionate, and linalyl butyrate), ketones (e.g., menthone, ionone, damascenone, and ethyl maltol), alcohols (e.g., geraniol, linalool, anethole, and eugenol), aldehydes (e.g., vanillin, benzaldehyde, and anisaldehyde), lactones (e.g., γ-undecalactone and γ-nonalactone), animal flavoring agents (e.g., musk, ambergris, civet, and castoreum), and hydrocarbons (e.g., limonene and pinene). One such substance may be used alone as a flavoring agent, or two or more may be used in combination in any proportions.

### 2) pH-Adjusting Agent

pH-adjusting agents are not limited, and examples include sodium carbonate, sodium hydrogencarbonate, potassium carbonate, potassium hydrogencarbonate, potassium phosphate, anhydrous sodium phosphate, sodium dihydrogenphosphate, and sodium citrate. Of these, sodium carbonate, potassium carbonate, or sodium dihydrogenphosphate is particularly preferred from the viewpoint of impact on the taste that the product exhibits. One such substance may be used alone as a pH-adjusting agent, or two or more may be used in combination in any proportions.

### 3) Sweetener

Examples of sweeteners include, although not limited, sugar alcohols, such as xylitol, maltitol, and erythritol; and substances such as acesulfame potassium, sucralose, and aspartame. Sugar alcohols are preferred from the viewpoint of taste adjustment. One sweetener may be used alone, or two or more may be used in combination in any proportions.

### 4) Bitterness Reducer

Bitterness reducers are not limited, but an example is soybean lecithin. Soybean lecithin is phospholipids, with specific examples including phosphatidylcholine, phosphatidylethanolamine, and phosphatidic acid. One such substance may be used alone as a bitterness reducer, or two or more may be used in combination in any proportions.

### 5) Humectant

Humectants are not limited, but examples include polyhydric alcohols, such as glycerine and propylene glycol. From the viewpoint of product storability, glycerine is preferred. One such substance may be used alone as a humectant, or two or more may be used in combination in any proportions.

### 6) White Coloring Agent

White coloring agents are not limited, but examples include particulate silicon dioxide, titanium dioxide, and calcium carbonate. From the viewpoint of the impact of taste on the product, particulate silicon dioxide is preferred. One such substance may be used alone as a white coloring agent, or two or more may be used in combination in any proportions.

### 7) Emulsifier or Surfactant

Emulsifiers (excluding polyglycerine fatty acid esters) are not limited, but examples include emulsifiers that are added to food. An example of an emulsifier is one or more selected from the group consisting of sucrose fatty acid esters, organic acid glycerine fatty acid esters, polyglycerine fatty acid esters, and lecithins. Examples of sucrose fatty acid esters include sucrose palmitate and sucrose stearate. Examples of organic acid glycerine fatty acid esters include succinic acid glycerine fatty acid esters and diacetyltartaric acid glycerine fatty acid esters. Examples of polyglycerine fatty acid esters include decaglycerine fatty acid esters. The percentage of emulsifiers in the composition is preferably an amount that makes the total percentage including the polyglycerine fatty acid ester described above fall within the ranges specified above as percentages of polyglycerine fatty acid esters.

### 8) Gelling Agent or Gelling Aid

The composition according to the present invention may contain a gelling agent other than the alginic acid compound. For use as the gelling agent, polysaccharides having a carboxyl group are preferred; for example, carrageenan, pectin, gum arabic, xanthan, gellan, and gum tragacanth are preferred. Carrageenan, pectin, and gellan, furthermore, are preferred because they easily gel in the presence of calcium ions and are capable of forming a crosslink structure by creating junction zones with carboxyl groups and cations. One such substance may be used alone, or two or more may be used in combination in any proportions.

An example of an ingredient that aids in gelation is calcium ions, and examples of their sources (gelling aids) include, although not limited, hydrogen halide salts (e.g., chloride), the citrate, the carbonate, the sulfate, the phosphate, and the lactate of calcium. Of these, calcium lactate, calcium chloride, or calcium phosphate is preferred because of small impact on taste, high solubility, and the pH after dissolution. In particular, calcium lactate is preferred. One such substance may be used alone, or two or more may be used in combination in any proportions.

Examples of ingredients that aid in gelation other than calcium ions include ions of metals such as magnesium, silver, zinc, copper, gold, and aluminum, with which the gelling agent can be bound via ionic bonds in the same manner as with calcium ions, and ions of cationic polymers. Examples of their sources (other gelling aids) include hydrogen halide salts (e.g., chloride), citrates, carbonates, sulfates, and phosphates of these metal ions and cationic polymers. One such substance may be used alone, or two or more may be used in combination in any proportions.

### (9) Characteristics

### 1) Flowability

Based on the flowability of the composition, the viscosity, adhesion to manufacturing devices, anticaking properties, and stickiness of the composition can be evaluated. The flowability is evaluated through a relative comparison of a shear stress value at a normal stress of 5.0 kPa at a measuring temperature of 22°C. The normal stress of 5.0 kPa is a pressure load expected to be applied to the composition under its own weight, for example during its manufacture, transport, or storage, as a condition under which the adhesion to manufacturing devices, caking, and stickiness of the composition can arise. The shear stress is preferably 4.15 kPa or more, more preferably 4.20 kPa or more, even more preferably 4.25 kPa or more, and preferably is 5.85 kPa or less, more preferably 5.80 kPa or less.

This shear stress of the composition at a normal stress of 5.0 kPa can be measured using a rheometer. For example, when the rheometer used is FT4 Powder Rheometer, manufactured by Freeman Technology Ltd., the shear stress is measured under the following measuring conditions.
- Measurement mode: standard program (25mm_shear_9kPa)
- Measuring temperature: 22°C
- Measuring humidity: 60%RH
- Measuring vessel: A cylindrical vessel with an inside diameter of 25 mm; volume, 10 ml
- Normal loads: 3-9 kPa

The raw materials of interest are each sieved (1.18-mm mesh), and the resulting fine and uniform particles are used as samples for measurement. Measurement is performed according to the procedure for the rheometer.

### 2) Particle Size

The composition is preferably composed of multiple solid granular pieces, but the size of the granular pieces is not limited. For a good feel in the mouth during use by the user, the ease of handling during manufacture, and controlled variations in quality, the composition is typically of a size where it passes through a sieve having a sieve aperture of 15 mm (< 15 mm) after being dried. Preferably, the dried composition is of a size where it passes through a sieve having a sieve aperture of 10 mm (< 10 mm), more preferably of a size where it passes through a sieve having a sieve aperture of 5 mm (< 5 mm), even more preferably of a size where it passes through a sieve having a sieve aperture of 3.2 mm (< 3.2 mm). For example, when all of the composition dried to a water content of 5% by weight or less has passed through a sieve with a sieve aperture of 3.2 mm, it indicates that the maximum particle size after drying of the composition is 3.2 mm or less. There is no need to set the lower limit to the particle size of the composition after drying, but typically, the particle size is 3 µm or more for the prevention of leakage from the pouches.

The method for preparing the dried composition described above is not limited as long as it allows for the reduction of the water content to 5% by weight or less, but an example is the method of preparing it by allowing the oral composition to stand for a predetermined duration under temperature conditions such as normal temperature or 70°C-80°C. The maximum particle size of the composition can be controlled as appropriate, for example by adjusting the particle size, water content, etc., of the ion-exchange resin with nicotine supported on it.

### 3) pH

The pH of the composition is not limited, but typically is 7.0 or higher because of impact on taste. Preferably, the pH is 7.5 or higher, more preferably 8.0 or higher. The pH, furthermore, is typically 10.0 or lower, preferably 9.5 or lower, more preferably 9.0 or lower. This pH is a measured value at 25°C.

The pH of the composition at a measuring temperature of 25°C can be measured using a pH analyzer (e.g., LAQUA F-72 with a flat ISFET pH electrode: manufactured by HORIBA, Ltd.), by adding 20 mL of water to 2 g of the composition, shaking the mixture for 10 minutes, and analyzing the supernatant. The calibration of the equipment is performed preferably by three-point calibration using a phthalate pH standard solution (pH 4.01), a phosphate pH standard equimolal solution (pH 6.86), and a tetraborate pH standard solution (pH 9.18) (all from Wako Pure Chemical Industries, Ltd.).

### 2. Oral Product

The oral product is used by taking it in the mouth. The form of the product can be as known, with examples including chewing tobacco, pouch, tablet, gum, film, and liquid forms. A chewing tobacco product refers to a product intended to be masticated for the mixing of nicotine contained in a composition with saliva and subsequent ingestion. A pouch product refers to a product that includes a sealed water-insoluble exterior material (also referred to as a pouch) and a composition (also referred to as a main material or filling) in it. Saliva can permeate through the pouch and dissolve ingredients in the composition contained in the pouch and then take the ingredients out through the pouch into the oral cavity. A tablet product refers to a product intended to be gradually dissolved or disintegrated in the mouth for the ingestion of ingredients in a composition, for example through the oral cavity or pharynx. Types of tablet products include, for example, troches, chewable tablets, and drops. A gum product refers to a product intended for the user to masticate them for the mixing of ingredients contained in a composition with saliva and subsequent absorption through the oral mucosa. Types of gum products include, for example, chewing gum and bubblegum. A film product refers to a product that adheres to the oral mucosa with the help of water, for example in saliva, when applied to the oral cavity. Through the mucosa, ingredients contained in a composition are ingested. A liquid product refers to a product intended to be applied into the oral cavity through spraying or dropwise addition for the ingestion of ingredients in a composition through the oral mucosa.

The size of the oral product is adjusted as appropriate according to the shape of the product. The amount of the alginic acid compound contained in each unit of the oral product, therefore, is prepared as appropriate.

When the oral product includes an exterior material and a main material, for example like pouches, a tablet product, or gum, it is preferred that the main material be the composition described above. The alginic acid compound, furthermore, can be contained in the exterior material, the main material, or both of them. In that case, the main material may be the composition or a composition equivalent to it, but preferably, the amount of the alginic acid compound is adjusted so that its total amount will be the desired amount per product.

In the present invention, oral products in pouch form, or oral pouch products, are particularly preferred. In the following, therefore, this type of product will be described as an example. An oral pouch product includes a main material (also referred to as filling) and an exterior material in which the main material is packaged (also referred to as a pouch).

### (1) Pouch

The pouch is not limited and can be a known one as long as it allows the filling to be packaged therein, is insoluble in water, and, at the same time, is permeable to liquids (e.g., water and saliva) and water-soluble ingredients in the filling. An example of a material for the pouch is cellulose-based nonwoven fabric, and commercially available nonwoven fabric may also be used. The pouch product can be produced by shaping a sheet made from such a material into a bag, packing the filling into the bag, and sealing the bag by means such as heat sealing.

The grammage of the sheet is not particularly restricted and typically is 12 gsm (g/m²) or more and 54 gsm or less, preferably 24 gsm or more and 30 gsm or less. The thickness of the sheet is not particularly restricted and typically is 100 µm or more and 300 µm or less, preferably 175 µm or more and 215 µm or less.

At least one of the inner surface or outer surface of the pouch may be partially coated with a water-repellent material. For use as the water-repellent material, a water-repellent fluoropolymer is suitable. Specifically, an example of a water-repellent fluoropolymer of this type is AsahiGuard^{®}, manufactured by Asahi Glass Co., Ltd. Water-repellent fluoropolymers are used to coat packaging materials for fat-containing foods and products, including confectionery, dairy products, ready-prepared foods, fast food, and pet food. Water-repellent fluoropolymers of this type, therefore, are safe even when applied to a pouch intended to be placed in the oral cavity. This water-repellent material is not limited to fluoropolymers; it may be a material having a water-repellent effect such as a paraffin resin, silicone resin, or epoxy resin.

The pouch can contain the alginic acid compound. For example, by applying a dispersion in which the alginic acid compound has been dispersed to the pouch, spraying such a dispersion onto the pouch, or impregnating the pouch with such a dispersion, a pouch containing the alginic acid compound can be prepared. Incorporating the alginic acid compound into the pouch makes it possible for the alginic acid to rapidly dissolve in saliva when the product comes into contact with saliva during its use, thereby allowing for the reduction of the uncomfortable feeling from the nonwoven fabric that occurs early in taking the product. In addition, incorporating the alginic acid compound into the pouch after mixing it with additives, such as a flavoring agent and a sweetener, makes it possible for the additives to be stably supported until the product is used yet immediately come into contact with saliva once the product is used. As a result, it becomes possible to further enhance palatability to users. The pouch, furthermore, may contain any ingredients. Examples of such ingredients include raw materials for adjusting flavor and taste, flavoring agents, additives, tobacco extract, and colorants. Moreover, the form in which these ingredients are contained is not limited, and examples include the form in which the ingredients are applied to the surface of the pouch or infiltrated into the pouch and, when the pouch is made of fibers, the form in which the ingredients are introduced into the fibers.

The appearance of the pouch is not limited either. The pouch may be nontransparent, translucent, or transparent. When the pouch is translucent or transparent, the filling is visible through it.

The size of the oral pouch product is not limited. For the size of the product before use, the lower limit to the longer sides may be 25 mm or more, 28 mm or more, 35 mm or more, or 38 mm or more. The upper limit, furthermore, may be 40 mm or less. The lower limit to the shorter sides may be 10 mm or more or 14 mm or more. The upper limit, furthermore, may be 20 mm or less or 18 mm or less. The percentage of the weight of the filling to the total weight of the oral pouch product is typically 80% by weight or more, although not limited. Preferably, the percentage is 85% by weight or more, more preferably 90% by weight or more. The percentage, furthermore, is typically 99% by weight or less, preferably 97% by weight or less, more preferably 95% by weight or less.

### (2) Filling

The amount of filling per unit of the oral pouch product is preferably 0.4-1.5 g. For the oral pouch product, the alginic acid compound can be contained in its exterior material, its filling, or both of them.

### (3) Alginic Acid Compound

The amount of the alginic acid compound contained per unit of the oral pouch product is preferably 0.02-1.2 g, more preferably 0.05-0.8 g.

### 3. Manufacturing Method

### (1) Manufacture of the Composition for Oral Product

As mentioned above, the alginic acid compound is prepared preferably through a step of reducing the molecular weight of a raw-material alginic acid compound having a higher molecular weight than the desired alginic acid compound by heating it for 50 minutes or more at 95-100°C under normal pressure. To achieve a good mouthfeel of the oral product, some amount of the alginic acid compound needs to be used. However, the raw-material alginic acid compound, which has a high molecular weight, improves the viscosity of the composition in proportion to its concentration. Consequently, the efficiency in the manufacture of the composition decreases. Of particular note is that the raw-material alginic acid compound can cause troubles during the manufacture of the composition, such as a reduced yield of the composition resulting from the adhesion of the composition or raw material, for example to a mixing vessel or transport device, and the occurrence of clogging inside devices resulting from reduced fluidity of the composition. Worse yet, the raw-material alginic acid compound causes uneven distribution of vegetable alkaloids, such as nicotine, because its ability to intensify binding in the composition often renders the composition nonuniform. In the present invention, however, a low molecular weight alginic acid compound as described above is used. These troubles, therefore, can be resolved, and the efficiency in the manufacture of the composition can be improved.

The composition is manufactured by any method, but preferably is manufactured through a step of mixing at least the alginic acid compound and optionally a substrate, a sugar alcohol, a polyglycerine fatty acid ester, and a release agent. The mixing can be carried out by introducing all raw materials into a mixer and mixing them.

In a preferred manufacturing method, nicotine, the alginic acid compound, a substrate, a sugar alcohol, a release agent, and optionally water and extra substances (e.g., a sweetener, a flavoring agent, and a humectant) are first mixed together to give a first mixture. Heating may be performed during this. The order of mixing of the raw materials, furthermore, is not limited; the materials may be put into the mixer and mixed together in any order or simultaneously, or solid raw materials may be mixed until uniform before adding liquid raw materials and further mixing. When the ease of operation is considered, the latter approach is preferred.

Then a polyglycerine fatty acid ester solution is sprayed onto the first mixture while the first mixture is stirred. Through this, the first mixture and the polyglycerine fatty acid ester are mixed together, giving a second mixture. Because the polyglycerine fatty acid ester will produce an anticaking effect, caking will be less likely to occur in subsequent steps. The solvent for the polyglycerine fatty acid ester solution is not limited as long as the polyglycerine fatty acid ester can be dissolved in it, but preferably is an alcohol solvent, such as ethanol. To prevent much of the solvent for the polyglycerine fatty acid ester solution from remaining in the second mixture, heating treatment may be applied during or after the end of the spraying of the polyglycerine fatty acid ester solution onto the first mixture.

After the preparation of the second mixture, furthermore, a treatment in which the second mixture is dried may be performed (drying step). After that, a treatment in which the mixture is cooled may be performed. The cooling may be natural cooling or may be performed using some cooling means (cooling step). By performing drying, the water content of the second mixture can be adjusted, for example to a desired value within the range of 5-60% by weight. This facilitates the adjustment of the water content of the composition.

To the mixture obtained as described above, an aqueous solution containing a pH-adjusting agent, a sweetener, such as acesulfame potassium, a flavoring agent, such as menthol, a bitterness reducer, such as soybean lecithin, and a humectant, such as glycerine, can optionally be added (additive addition step). These additives may be added as solids or may be added as aqueous solutions, in which the additives have been dissolved in water. When added as aqueous solutions, the additives may be pre-dissolved in a predetermined amount of water before addition so that the final water content of the oral product can be achieved.

### (2) Manufacture of the Oral Product

By processing the composition by a known method, the composition can be made into the oral product. In the following, the method will be described by taking the example of an oral pouch product.

By packaging the composition with the exterior material, the oral pouch product can be manufactured (packaging step). The method for packaging the composition is not limited, and known methods can be applied. For example, known methods such as the method of putting the composition into a bag-shaped nonwoven fabric and then sealing the bag can be used. In the packaging step, desired additional water may be added after sealing (hydration step). For example, when the percentage of water in the final composition is 50% by weight and when the percentage of water in the packed composition is 15% by weight, water equivalent to the remaining 35% by weight is added.

### EXAMPLES

### [Manufacturing Example 1]

A high molecular weight alginic acid compound is dissolved in water to form an aqueous solution. The aqueous solution is heated for 60-300 minutes at 95°C under normal pressure to give a low molecular weight alginic acid compound. The weight-average molecular weight (measurement by GPC) of the resulting low molecular weight alginic acid compound is approximately 400,000-900,000.

### [Manufacturing Example 2] Addition of Organic Acid

An aqueous solution is prepared in which a high molecular weight alginic acid compound has been dissolved in water and in which a 1.0 M aqueous solution of citric acid has been additionally mixed. The aqueous solution is heated for 30-300 minutes at 95°C under normal pressure to give a low molecular weight alginic acid compound. The weight-average molecular weight (measurement by GPC) of the resulting low molecular weight alginic acid compound is approximately 13,000-900,000.

### [Example 1]

A composition for an oral product is produced by mixing the ingredients specified below using a mixer.
Nicotine, 2 parts by weight
The alginic acid compound of Manufacturing Example 1, 10.5 parts by weight
Maltitol, 37.8 parts by weight
Cellulose, 22.7 parts by weight
Sodium phosphate, 14.3 parts by weight
Polyglycerine fatty acid ester, 10.3 parts by weight
Silica, 2.1 parts by weight
Acesulfame K, 0.3 parts by weight

### [Example 2] Oral Pouch Product

An oral pouch product is manufactured by collecting 0.7 g of the composition obtained in Example 1 and packaging it in a known pouch.

### [Example 3] Oral Film Product

A composition for an oral product is produced by stirring and mixing the ingredients specified below with simultaneous homogenization using a mixer under heated conditions at 80°C.
Nicotine, 10 parts by weight
Aqueous solution of the alginic acid compound of Manufacturing Example 1 (20% by weight aqueous solution), 43.5 parts by weight
Polyglycerine fatty acid ester, 3.1 parts by weight
Sodium dihydrogenphosphate, 10.2 parts by weight
Potassium phosphate, 20.5 parts by weight
Maltitol, 5 parts by weight
Silica, 2.1 parts by weight
Flavoring agent, 5.6 parts by weight

The composition obtained in the foregoing is evenly spread (to a thickness of 1 mm) onto a casting substrate, and the substrate is dried for 20 minutes at 60°C in a drying cabinet. The water content of the product after drying is 8 parts by weight. The dried film is cut by a known cutting method, and thereby individual oral films are manufactured.

## Claims

1. A composition for an oral product, the composition comprising an alginic acid compound having a weight-average molecular weight of 10,000-900,000 and selected from the group consisting of alginic acid, salts thereof, esters thereof, and combinations of the foregoing.

2. The composition according to claim 1, wherein an amount of the alginic acid compound in the composition is 1-99% by weight.

3. The composition according to claim 1 or 2, further comprising a vegetable alkaloid.

4. An oral product comprising the composition according to any of claims 1 to 3.

5. An oral product comprising an exterior material and a main material, wherein:
the exterior material or the main material contains an alginic acid compound having a weight-average molecular weight of 10,000-900,000 and selected from the group consisting of alginic acid, salts thereof, esters thereof, and combinations of the foregoing.

6. The oral product according to claim 5, wherein the exterior material contains the alginic acid compound.

7. The oral product according to claim 4, wherein the oral product is in chewing tobacco, pouch, tablet, gum, film, or liquid form.

8. The oral product according to claim 5 or 6, wherein the oral product is in pouch or gum form.

9. A method for manufacturing the composition according to any of claims 1 to 3, the method including:
a step of preparing the alginic acid compound by heating a raw-material alginic acid compound having a higher molecular weight than the alginic acid compound for 50 minutes or more at 95-100°C under normal pressure.
